# EUROPEAN PATENT APPLICATION

(11) **EP 2 368 566 A1**
(43) Date of publication of application: **28.09.2011**
(21) Application number: 11171346.7
(22) Date of filing: 13.07.2007
(51) Int. Cl.: A61K 38/20, A61K 31/4412, A61K 31/404, A61P 35/00

(54) **Interleukin 21 and tyrosine kinase inhibitor combination therapy**

(30) Priority: 13.07.2006 US 807256 P
(62) Divisional of application: 07812925.1
(71) Applicant: ZymoGenetics, Inc., Seattle, Washington 98102 (US)
(72) Inventor: Sivakumar, Pallavur V., Seattle, WA Washington 98117 (US); Hausman, Diana F., Seattle, WA Washington 98102 (US); Hughes, Steven D., Kenmore, WA Washington 98028 (US); Sievers, Eric, Seattle, WA Washington 98112 (US); Miller, Dennis M., Woodinville, WA Washington 98072 (US)
(74) Representative: Griffin, Philippa Jane

(57) **Abstract**

There is provided a composition comprising IL-21 polypeptide and a composition comprising a tyrosine kinase inhibitor, for use in treating cancer selected from renal cell carcinoma, metastatic melanoma, or hepatocellular carcinoma, wherein said use comprises co-administration of said compositions to a patient.

## Description

### BACKGROUND OF THE INVENTION

Interleukin-21 (IL-21) is a type I cytokine produced endogenously by activated CD4+ T cells as a polypeptide of 133 amino acids with an approximate molecular weight of 15.6 kDa (Parrish-Novak, et al., Nature, 408:57-63, 2000). Its sequence, protein structure, and gene structure place it in the IL-2 family of cytokines, with greatest similarity to IL-2 and IL-15. Like those cytokines, IL-21 recruits the common cytokine receptor γ chain (γc) as a component of its receptor complex, which also includes an IL 21-specific receptor protein, IL-21R (Parrish-Novak, et al., 2000). Expression of IL-21R is primarily restricted to lymphoid tissues and peripheral blood mononuclear cells. Under normal physiological conditions, IL-21 is likely sequestered within the local area of production.

IL-21 has been administered as a monotherapy to patients with renal cell carcinoma and metastatic melanoma in clinical trials (Redman et al., J. Clin. Oncology, 23 (16; Suppl 1.) 166S, 2005; McArthur et al., Eur. J. Cancer, Suppl. 3 (2):148,2005.) Combination treatment with IL-21 and rituximab has been demonstrated to enhance the anti-tumor effect over rituximab alone in in vitro and in vivo models of B cell lymphoma (Hughes et al., Blood 104 (11 Part 1): 394A, 2004.)

Tyrosine kinases are enzymes that catalyze the transfer of the γ phosphate group from the adenosine triphosphate to target proteins. Tyrosine kinases can be classified as receptor and nonreceptor protein tyrosine kinases. They play an essential role in diverse normal cellular processes, including activation through growth receptors and affect proliferation, survival and growth of various cell types. Additionally, they are thought to promote tumor cell proliferation, induce anti-apoptotic effects and promote angiogenesis and metastasis. In addition to activation through growth factors, protein kinase activation through somatic mutation is a common mechanism of tumorigenesis. Some of the mutations identified are in B-Raf kinase, FLt3 kinase, BCR-ABL kinase, _{c}-KIT kinase, epidermal growth factor (EGFR) and PDGFR pathways. The Her2, VEGFR and c-Met are other significant receptor tyrosine kinase (RTK) pathways implicated in cancer progression and tumorigenesis. Because a large number of cellular processes are initiated by tyrosine kinases, they have been identified as key targets for inhibitors.

Tyrosine kinase inhibitors (TKIs) are small molecules that act inside the cell, competing with adenosine triphosphate (ATP) for binding to the catalytic tyrosine kinase domain of both receptor and non-receptor tyrosine kinases. This competitive binding blocks initiation of downstream signaling leading to effector functions associated with these signaling events like growth, survival, and angiogenesis. Using a structure and computational approach, a number of compounds from numerous medicial chemistry combinatorial libraries was identified that inhibit tyrosine kinases. BAY 43-9006 (sorafenib, Nexavar®) and SU 11248 (sunitinib, Sutent®) are two such TKIs that have been recently approved for use in metastatic renal cell carcinoma (RCC). A number of other TKIs are in late and early stage development for treatment of various types of cancer.

Although the FDA has approved two new orally active tyrosine kinase inhibitors for the treatment of advanced kidney cancer, curative therapy remains elusive. At present, only high-dose interleukin 2 (IL-2) has elicited durable complete responses in approximately 7% of selected patients. Because high dose IL-2 is associated with marked clinical toxicities and the durable response rate is relatively low, its use has declined over the past two decades. Despite recent innovations, advanced kidney cancer remains an unmet medical need. The present invention provides compositions and methods for these unmet medical needs in kidney cancer and well as other indications using a combination of IL-21 and a TKI.

These and other aspects of the invention will become apparent to those persons skilled in the art upon reading the details of the invention as more fully described below.

### SUMMARY OF THE INVENTION

Within one aspect, the present invention provides a method of treating renal cell carcinoma or metastatic melanoma comprising co-administering to a patient a composition comprising IL-21 polypeptide and a composition comprising a tyrosine kinase inhibitor.

In one aspect, the present invention provides a method of treating renal cell carcinoma comprising co-administering to a patient a composition comprising an IL-21 polypeptide and a composition comprising sutinitib.

In one aspect, the present invention provides a method of treating renal cell carcinoma comprising co-administering to a patient a composition comprising an IL-21 polypeptide and a composition comprising sorafenib.

In certain embodiments of the methods, the IL-21 composition is administered on a 5/9/5 schedule until disease progression. In other embodiments of the methods, the IL-21 composition is administered from 1 to 3 times weekly. In other embodiments of the methods, the IL-21 composition is administered comcomitantly with the sorafenib composition. In certain other embodiments of the methods, the sorafenib composition is administered at 800 mg daily.

### DESCRIPTION OF THE INVENTION

Prior to setting forth the invention in detail, it may be helpful to the understanding thereof to define the following terms:

The term "cancer" or "cancer cell" is used herein to denote a tissue or cell found in a neoplasm which possesses characteristics which differentiate it from normal tissue or tissue cells. Among such characteristics include but are not limited to: degree of anaplasia, irregularity in shape, indistinctness of cell outline, nuclear size, changes in structure of nucleus or cytoplasm, other phenotypic changes, presence of cellular proteins indicative of a cancerous or pre-cancerous state, increased number of mitoses, and ability to metastasize. Words pertaining to "cancer" include carcinoma, sarcoma, tumor, leukemia, lymphoma, polyp, neoplasm, and the like.

The term "co-administration" is used herein to denote that an IL-21 polypeptide or protein and a TKI may be given concurrently or at different times of a treatment cycle. The co-adminstration may be a single co-administration of both IL-21 and TKI or multiple cycles of co-administration, where both IL-21 and a TKI are both given, at least once, within a three month period. Co-administration need not be the only times either IL-21 or the TKI is administered to a patient and either agent may be administered alone or in a combination with therapeutic agents other than IL-21.

The term "combination therapy" is used herein to denote that a subject is administered at least one therapeutically effective dose of an IL-21 composition ("IL-21") and a TKI.

The term "level" when referring to immune cells, such as NK cells, T cells, in particular cytotoxic T cells, B cells and the like, denotes increased level as either an increased number of cells or enhanced activity of cell function and decreased level as a decreased number of cells or diminished activity of cell function.

The term "optimal immunological response" refers to a change in an immunological response after administration of IL-21 or the IL-21 + TKI combination over that seen when the TKI alone is administered, and can be (1) an increase in the numbers of activated or tumor specific CD8 T cells, (2) an increase in the numbers of activated or tumor specific CD8 T cells expressing higher levels of granzyme B or perforin or IFNγ, (3) upregulation of Fcγ receptor (CD16, CD32, or CD64) on Nk cells, monocytes, or neutrophils, (4) an increase in soluble CD25 in the serum, (5) reduction in serum level of proteins liberated by tumor cells (see, Taro et al., J. Cell Physiol. 203(1):1-5, 2005), for example, carcinoembryonic antigen (CEA), IgG, CA-19-9, or ovarian cancer antigen (CA125), (6) an increase in the numbers of NK cells expressing higher levels of granzyme B, perforin or IFNγ, (7) increase in the levels of activation cytokines such as IL-18, IL-15, IFNγ and chemokines that enable homing of effector cells to the tumor, such as IP-10, RANTES, IL-8, MIP1a or MIP1b, (8) an increase in the numbers of activated macrophages in the periphery or at the tumor site, where activation can be detected by expression of increased MHC class I or Class II, production ofIL-15, IL-18, IFNγ, or IL-21, or (9) macrophage activity as indicated by decline in red blood cell count (severity of anemia).

The term "progression free survival" (PFS) is used herein to be defined as the time from randomization until objective tumor progression or death. For non-randomized studies, PFS is defined as the time from first dose of study medication until objective tumor progression or death.

The term "synergistic" is used herein to denote a biological or clinical activity of two or more therapeutic agents that when measured is greater than either agent alone.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Methods and materials similar or equivalent to those described herein can be used in the practice or testing of the present invention. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a polymorphism includes a plurality of such polymorphisms, reference to "a nucleic acid molecule" includes a plurality of such nucleic acid molecules, and reference to "the methods" includes reference to one or more methods, method steps, and equivalents thereof known to those skilled in the art, and so forth.

The present invention is directed to the use of IL-21 in combination with a tyrosine kinase inhibitor (TKI) in treatment of diseases and disorders in which inhibition of phosphorylation via TK inhibition and modulation of immune function play a clinically beneficial role. These diseases and disorders include, but are not limited to, cancer, infection and autoimmune disease.

While not intending to be bound by any theory, there are certain cases in which the combination of IL-21 and a TKI can be used therapeutically where inhibition of the transfer of the γ phosphate group from the adenosine triphosphate to target proteins and blocking initiation of downstream signaling and stimulation of an immune response play an important role. Treatment of such diseases and disorders includes inhibition of tumor cell proliferation, induction of anti-apoptotic effects and inhibition angiogenesis and metastases. In addition to activation through growth factors, protein kinase activation through somatic mutation is a common mechanism of tumorigenesis. Preclinical evaluation of the combination of TKIs and IL-21 suggests that the TKIs, when used at concentrations simulating therapeutic exposure, do not inhibit IL-21 or immune effector functions in vitro. Further, IL-21, in combination with TKI has been shown to have additive effects in preclinical models.

Three types of cancer that have shown sensitivity to treatment with either IL-21 protein or a TKI in the clinic are renal cell carcinoma, metastatic melanoma and hepatoceullular carcinoma. Patient outcomes in RCC and other cancers and diseases can be improved through use of a combination of TKIs and immunotherapic agent. To date, immunotherapy (with high-dose aldesleukin) is the only treatment associated with durable complete responses (CRs) in RCC and metastatic melanoma (MM) patients. Therefore, combining these two classes of therapeutic agents will lead to additive or possibly even synergistic benefits for patients.

Most TKIs are thought to inhibit growth of tumors through direct inhibition of the tumor cell or through inhibition of angiogenesis. IL-21, in contrast enhances immune activation against tumor cells. Combination of these two agents provides additive or synergistic effects through attacking the cancer via multiple, but independent pathways.

Moreover, certain TKIs affect signaling through the VEGF family receptors, including sorafenib and sunitinib. VEGF has been shown to be both angiogeneic and affect the immune system. In vitro studies have demonstrated VEGF can inhibit dendritic cell-mediated stimulation of antigen-specific T cells (Laxmanan et al., BBRC 334(1):193-8, 2005), as well as in vivo studies in which administration of anti-VEGF antibody and peptide-pulsed dendritic cells has been associated with increased antitumor effects in mice (Gabrilovich et al., Clin Can Res. 5(10):2963-70, 1999). Therefore, use of a combination treatment comprising IL-21 along with a TKI that inhibits VEGF may enhance immune responses in patients.

TKIs affect tumor growth by a direct inhibition of the tumor or through effects on the microenvironment of the tumor (angiogenesis). TKI-mediated inhibition of tumor death may lead to increased antigen-presentation of dead tumor cells by antigen presenting cells (APCs), like macrophages and dendritic cells. IL-21 may then enhance T cell activity specific for these tumor antigens presented by APCs.

In some cases TKIs have been shown to activate functions of dendritic cells and other innate immune cells, like NK cells. This has been recently reported in animal models for imatinib. Imatinib is a TKI that has shown to enhance killer activity by dendritic cells and NK cells) for review, see Smyth et al, NEJM 354:2282, 2006). IL-21 may enhance activity of these NK cells and immune response mediated by dendritic cells.

Sorafenib, a novel biaryl urea was initially discovered in a large-scale screen to identify inhibitors of RAF kinase. Further characterization has shown that sorafenib is able to potently inhibit multiple tyrosine kinase pathways including c-Raf, b-raf, VEGFR2, VEGFR3, PDGFRβ, c-kit and flt3 (Wilhelm et al , Cancer Res. 64:7099: 2004). IC50 for inhibition of most of these kinases have been demonstrated in the nM range (6-250nM). In vitro, sorafenib is able to inhibit proliferation of multiple tumor cell lines. In vivo, therapeutic oral delivery of sorafenib at doses ranging from 10-60 mg/kg daily was shown to significantly inhibit growth of various xenograft tumors in nude mice (Wilhelm et al, 2004, supra). These include colon, breast, NSCLC, pancreatic and other solid tumors. Mechanism of sorafenib is thought to be mediated anti-tumor effects by a combination of direct anti-tumor activity by inhibiting Raf-kinase and through inhibiting angiogenesis mediated by the VEGF and PDGF pathways. Sorafenib is described in WO 00/41698.

Sorafenib is reported to inhibit growth of the mouse renal cell carcinoma cell line RenCa in vivo in both sub-cutaneous and orthotpic models (Schoffski et al, Annals of Oncology 17(3):450-6, 2006). BAY 43-9006 (sorafenib) inhibited ectopic (s.c.) and orthotopic growth of a murine model of renal adenocarcinoma (RenCa) predominately through inhibition of tumor angiogenesis (96th Annual Meeting of the American Association for Cancer Research; April 16-20, Anaheim, CA. Abstract 5831, 2005.). A dose dependent decrease in tumor growth has been reported ranging from 30% inhibition of tumor growth at 7.5 mg/kg daily to 84% inhibition at 60 mg/kg daily. The mechanism action was by inhibiting angiogenesis.

Sunitinib is an oral oxindole TK inhibitor, with selective multi-targeted inhibition of VEGFR1, VEGFR2, VEGFR3, PDGFRα, PDGFRβ, Flt3 and c-kit kinases. Sunitinib has also recently been shown to inhibit the RET kinase pathway. No data are currently available on it's ability to inhibit the Raf kinase pathway. In vitro, sunitinib was able to inhibit activation through multiple kinases with an IC50 of 4-70nM (Mendel et al, Clin Can Res 9:327, 2003). In vivo, oral delivery of sunitinb is able to inhibit growth of human tumor xenografts in mice at doses ranging from 20-80 mg/kg/day. Tumor growth inhibition was demonstrated in multiple tumor types including colon, NSCLC, gliomas, melanoma, breast and epidermoid tumors. In some cases, regression of tumors was observed after therapeutic delivery (Mendel et al, 2003, ibid.). Mechanism of sunitinib-induced tumor regression was demonstrated to be due to inhibition of angiogenesis in vivo. Sunitinib also shows potent anti-tumor activity in vitro and in vivo against AML lines by blocking signaling through the flt3 kinase (O'Farrell et al, Blood 101:3597, 2003) and has demonstrated potent anti-tumor activity in combination with radiotherapy (Schueneman et al, Cancer Res, 63:4(4009, 2003). Sunitinib is described in U.S. Patent Serial No. 6,573,293.

Other TKIs include, but are not limited to: Imatinib mesylate (Gleevec®, Novartis); Gefitinib (Iressa®, AstraZeneca); Erlotinib hydrochloride (Tarceva®, Genentech); Vandetanib (Zactima®, AstraZeneca), Tipifarnib (Zarnestra®, Janssen-Cilag); Dasatinib (Sprycel®, Bristol Myers Squibb); Lonafarnib (Sarasar®, Schering Plough); Vatalanib succinate (Novartis, Schering AG); Lapatinib (Tykerb®, GlaxoSmithKline); Nilotinib (Novartis); Lestaurtinib (Cephalon); Pazopanib hydrochloride (GlaxoSmithKline); Axitinib (Pfizer); Canertinib dihydrochloride (Pfizer); Pelitinib (National Cancer Institute, Wyeth); Tandutinib (Millennium); Bosutinib (Wyeth); Semaxanib (Sugen, Taiho); AZD-2171 (AstraZeneca); VX-680 (Merck, Vertex); EXEL-0999 (Exelixis); ARRY-142886 (Array BioPharma, AstraZeneca); PD-0325901 (Pfizer); AMG-706 (Amgen); BIBF-1120 (Boehringer Ingelheim); SU-6668 (Taiho); CP-547632 (OSI); (AEE-788 (Novartis); BMS-582664 (Bristol-Myers Squibb); JNK-401 (Celgene); R-788 (Rigel); AZD-1152 HQPA (AstraZeneca); NM-3 (Genzyme Oncology); CP-868596 (Pfizer); BMS-599626 (Bristol-Myers Squibb); PTC-299 (PTC Therapeutics); ABT-869 (Abbott); EXEL-2880 (Exelixis); AG-024322 (Pfizer); XL-820 (Exelixis); OSI-930 (OSI); XL-184 (Exelixis); KRN-951 (Kirin Brewery); CP-724714 (OSI); E-7080 (Eisai); HKI-272 (Wyeth); CHIR-258 (Chiron); ZK-304709 (Schering AG); EXEL-7647 (Exelixis); BAY-57-9352 (Bayer); BIBW-2992 (Boehringer Ingelheim); AV-412 (AVEO); YN-968D1 (Advenchen Laboratories); Midostaurin (Novartis); Perifosine (AEterna Zentaris, Keryx, National Cancer Institute); AG-024322 (Pfizer); AZD-1152 (AstraZeneca); ON-01910Na (Onconova); and AZD-0530 (AstraZeneca).

The optimal IL-21 dosing regimen will provide the most beneficial and sustainable stimulation of immune cells. For tumors in which IL-21 it believed to have no direct action on the tumor cells, such as MM and RCC, maximal exposure of tumor cells to IL-21 is not a goal of therapy. The potential benefit of IL-21 therapy is achieved through activating the immune system. An IL-21 treatment cycle is a regimen in which dosing with IL-21 and a rest period is completed. The co-adminstration may be a single co-administration of both IL-21 and TKI or multiple cycles of co-administration, where both IL-21 and a TKI are both given, at least once, within a three month period. Treatment cycles can be repeated until no further clinical benefit is foreseen or toxicity becomes unacceptable. In one embodiment, a treatment cycle for IL-21 is defined as cycles defmed as 5 days of treatment followed by a rest period. In certain embodiments, a 9 day period of rest (no IL-21 treatment), with an optional week of rest (5/9/5). In another embodiment, the rest period may be extended to 16 days. The five-day dosing period is believed to provide clinically significant pharmacological effects of rIL-21, and the 9-16 day dose-free period is used to normalize parameters prior to initiating a new treatment cycle. The 5/9/5 dosing regimen was demonstrated to be efficacious in preclinical tumor models (Hughes, et al., J. of Clinical Oncol. 22 (14S):187S, 2004).

An IL-21 composition may be a mature polypeptide, fragment thereof, fusion or conjugate that demonstrates IL-21 biological activity. An IL-21 composition could also include a buffer, for example, histidine, citrate or succinate buffers may be used. Tonicity adjusters such as mannitol, sorbitol, sodium chlorine or glycine can be used. Other excipients may also be included in the compositions of the present invention. For example, acceptable excipients include disaccharides, such as trehalose and sucrose as stabilizers; polyethylene glycol as a stablizer or wetting agent; surfactants, such as tween 20, tween 80 or triton-X-100 as a stablizer or wetting agent; or other bulking agents, such as glycine, hydroxyethyl starch. One or more preservatives may also be included in the compositions of the present invention, particularly in those compositions packaged for multiple use. Preservatives that can be used within the present invention include those commonly used in pharmaceutical preparations, such as methylparaben, propylparaben, benzyl alcohol, m-cresol, ethylmercurithiosalycilate, phenol, thimerosal, and the like.

The IL-21 composition is given by injection, either by intravenous (IV), or intramuscular (IM) or subcutaneous (SC.) routes of administration. The present invention provides for IL-21 compositions wherein each dose is in a range of about 10 µg/kg to 500 µg/kg. In certain embodiments, the IL-21 dose is in the range of 10 to 300 µg/kg. In other embodiments, the dose will be 10-30, 30-50, 50-75, or 75-100 µg/kg from once to five times weekly. Thus, in one embodiment, the present invention provides methods for administering an IL-21 composition on a 5/9/5 schedule until there is disease progression. In another embodiment, the present invention provides methods where the IL-21 composition is administered once weekly. In other embodiments, the IL-21 composition is administered 2, 3, or 4 times weekly.

Sorafenib dose is administered as 400 mg, twice daily for a maximum daily dose of 800 mg. Treatment continues until the patient is no longer clinically benefiting from therapy or until unacceptable toxicity occurs. Management of suspected adverse drug reactions may require temporary interruption and/or dose reduction. The dose may be reduced to 400 mg once daily, and additional dose reduction may be a single 400 mg dose every other day (see prescribing information for Nexavar® by Bayer Pharmaceuticals, West Haven, CT.) However, IL-21 may be used in combination with sorafenib with other approved dosing regimens. In certain embodiments of the present invention, the IL-21 composition is given concomitantly with the sorafenib composition.

Sunitinib is administered as a 50 mg dose once daily, on a schedule of four weeks on treatment followed by two weeks off treatment (see prescribing information for Sutent® by Pfizer, New York, NY.) Sunitinib may also be given at 37.5 mg daily without time off treatment or using any other approved dosing regimen.

IL-21 can be given concomitantly with sorafenib or sunitinib. In one embodiment, the general treatment strategy is to deliver ongoing cycles of IL-21 in the 5/9/5 regimen comcomitantly with a TKI until unacceptable toxicity, tumor progression or achievement of complete remission.

The following examples are given to illustrate representative embodiments of the invention and should not be considered as limiting the scope of the invention.

### EXAMPLES

### Example I

A dose response was done to study the effects of sorafenib and sunitnib in the RenCa.2 mouse renal cell carcinoma model. Sorafenib was maximally effective in mediating tumor clearance at doses ranging from 10 mg/kg-60 mg/kg but was partially effective at doses below 10 mg/kg. In contrast orally administered sunitnib was maximally effective only at doses above 40 mg/kg.

Two experiments were designed to address whether mIL-21, when concurrently administered with sorafenib had additive effects in the RenCa.2 model of RCC. In the first experiment, 25 µg mIL-21 was administered concurrently with sub-maximal 2 mg/kg sorafenib dose. As shown in Table 1, mIL-21 alone at this dose was partially effective in inhibiting tumor growth as was 2 mg/kg sorafenib. Concurrent administration of mIL-21 and sorafenib showed better inhibition of tumor growth than either drug alone (p=0.007 on Day 21), suggesting that IL-21 and sorafenib had additive effects in this model.

In the second experiment, mice were concurrently administered 50 µg mIL-21 or a maximal 60 mg/kg sorafenib dose alone or a combination of the two reagents. Survival of mice receiving the treatments were monitored over 35 days. As shown in Table 2, mice that did not receive any treatment did not survive past Day 22. Mice receiving mIL-21 alone showed only 10% survival by Day 35 whereas mice receiving 60 mg/kg sorafenib showed 50% survival at this time. In contrast, mice receiving both mIL-21 and sorafenib showed 100% survival at Day 35. This demonstrates that IL-21, in combination with high dose sorafenib had a survival advantage over mice receiving IL-21 or sorafenib alone, indicating additive or synergistic activity.

A. Concurrent administration of mIL-21 and sub-maximal sorafenib dose additive inhibition of RenCa.2 tumor growth in mice:

To test if concurrent administration of mIL-21 and sorafenib have additive effects on tumor growth in mice, groups of mice were injected s.c with the RenCa.2 tumor on Day 0. Mice were then injected with 25 µg mIL-21 or 2 mg/kg sorafenib alone or in combination. Tumor volume was monitored 3X/week for 3 weeks. Mice injected with combination of both mIL-21 and sorafenib showed significantly smaller tumors compared to mice injected with control reagent or with either mIL-21 or sorafenib alone, suggesting that concurrent administration of these two reagents has additive effects in this model.

Ten-week old female BALB/c mice (Charles River Laboratories) were injected s.c. on the right flank with 0.1 x 10⁶ RenCa.2 cells on Day 0. Starting day 3, groups of mice (n=10/group) were given no treatment (Group 1) or were injected i.p. with 25 µg mIL-21 (Group 2) alone for 5 days (Group 1), oral gavage of 2 mg/kg sorafenib alone for 10 days (Group 3) or the combination of the two reagents (Group 4). Tumor growth was monitored 3X/week for 3 weeks using caliper measurements. Tumor volume is calculated using the formula ½*(B)2*L (mm3).

Mice injected with combination of mIL-21 and sorafenib showed significantly smaller tumors compared to mice injected with either mIL-21 alone or sorfaenib alone, suggesting that combining these two reagents has additive effects in this model (Table 1, p=0.007 on Day 21 compared to mIL-21 alone or sorafenib alone, Student's t-test).

B. Concurrent administration of mIL-21 and high dose sorafenib dose has a survival advantage to mice bearing RenCa.2 tumors.

To test if concurrent administration of mIL-21 and high dose sorafenib have additive effects on tumor growth in mice, groups of mice were injected s.c with the RenCa.2 tumor on Day 0. Mice were then injected with 50 µg mIL-21 or 60 mg/kg sorafenib alone or in combination. Survival of mice were monitored over 35 days. All mice injected with combination of both mIL-21 and sorafenib survived at Day 35 compared to mice injected with control reagent or with either mIL-21 or sorafenib alone, suggesting that concurrent administration of these two reagents has additive effects in this model.

Ten-week old female BALB/c mice (Charles River Laboratories) were injected s.c. on the right flank with 0.1 x 10⁶ RenCa cells on Day 0. Starting day 5, groups of mice (n-10/group) were given no treatment (Group 1) or were injected i.p. with 50 µg mIL-21 (Group 2) + sorafenib vehicle for 5 days followed by 9 day rest, followed by 5 days treatment (Group 4), oral gavage of 60 mg/kg sorafenib alone for 10 days or the combination of the two reagents (Group 3). Tumor growth was monitored 3X/week for 3 weeks using caliper measurements. Survival of mice were monitered over 35 days. Tumor bearing mice were terminated as per IACUC policies with regard to maximal tumor size (1200mm³) or weight loss of >= 20% of initial body weight.

As shown in Table 2, mice that did not receive any treatment did not survive past Day 22. Mice receiving mIL-21 alone showed only 10% survival by Day 35 whereas mice receiving 60 mg/kg sorafenib showed 50% survival at this time. In contrast, mice receiving both mlL-21 and sorafenib showed 100% survival at Day 35. This shows that IL-21, in combination with high dose sorafenib had a survival advantage over mice receiving IL-21 or sorafenib alone (p<0.0001, Kaplein-Meier survival test).

**Table 1**

| Group # | Treatment groups | Tumor volume mm3 + SD(Day 21) | p-value |
|---|---|---|---|
| 1 | No treatment | 932.2 + 423.8 | |
| 2 | mlL-21 + sorafenib vehicle | 687.5 + 399 | |
| 3 | 2 mg/kg sorafenib + mIL-21 vechicle | 790.3 + 398.8 | |
| 4 | 2 mg/kg sorafenib + mIL-21 | 370.9 + 194.7 | 0.007 vs Gps 2 and 3 |

**Table 2**

| Group # | Treatment groups | % mice surviving at Day 35 | P -value |
|---|---|---|---|
| 1 | No treatment | 0 | |
| 2 | mIL-21 + sorafenib vehicle | 10 | |
| 3 | 60 mg/kg sorafenib + mIL-21 vehicle | 50 | |
| 4 | 60 mg/kg sorafenib + mIL-21 | 100 | <0.0001 |

### Example 2

There are two different types of primary liver cancer. The most common kind is called hepatoma or hepatocellular carcinoma (HCC), and arises from the main cells of the liver (the hepatocytes). This type is usually confined to the liver, although occasionally it spreads to other organs. Infection with either the hepatitis B or hepatitis C virus can lead to liver cancer, and can also be the cause of cirrhosis, which increases the risk of developing hepatoma. The hepatocellular carcinoma may or may not be associated with an hepatitis (e.g., hepatitis A, hepatitis B, hepatitis C and hepatitis D) infection.

The effects of a IL-21 composition in combination with a TKI on tumor response can be evaluated in a hepatocellular carcinoma transgenic mouse model, which includes the overexpression of transforming growth factor-α (TFG-α) alone (Jhappan et al., Cell, 61:1137-1146 (1990); Sandgren et al., Mol. Cell Biol., 13:320-330 (1993); Sandgren et al., Oncogene, 4:715-724 (1989); and Lee et al., Cancer Res., 52:5162:5170 (1992)) or in combination with c-*myc* (Murakami et al., Cancer Res., 53:1719-1723 (1993), mutated H-ras (Saitoh et al., Oncogene, 5:1195-2000 (1990)), hepatitis B viral genes encoding HbsAg and HBx (Toshkov et al., Hepatology, 20:1162-1172 (1994) and Koike et al., Hepatology, 19:810-819 (1994)), SV40 large T antigen (Sepulveda et al., Cancer Res., 49:6108-6117 (1989) and Schirmacher et al., Am. J. Pathol.,139:231-241 (1991)) and FGF19 (Nicholes et al., American Journal of Pathology, 160(6):2295-2307 (June 2002)).

### CLAUSES

1. A method of treating renal cell carcinoma or metastatic melanoma comprising co-administering to a patient a composition comprising IL-21 polypeptide and a composition comprising a tyrosine kinase inhibitor.
2. A method of treating renal cell carcinoma comprising co-administering to a patient a composition comprising IL-21 polypeptide and a composition comprising sutinitib.
3. A method of treating renal cell carcinoma comprising co-administering to a patient a composition comprising IL-21 polypeptide and a composition comprising sorafenib.
4. The method of Clause 1, wherein the IL-21 composition is administered on a 5/9/5 schedule until disease progression.
5. The method of Clause 2, wherein the IL-21 composition is administered on a 5/9/5 schedule until disease progression.
6. The method of Clause 3, wherein the IL-21 composition is administered on a 5/9/5 schedule until disease progression.
7. The method of Clause 1, wherein the IL-21 composition is administered from 1 to 3 times weekly.
8. The method of Clause 2, wherein the IL-21 composition is administered from 1 to 3 times weekly.
9. The method of Clause 3, wherein the IL-21 composition is administered from 1 to 3 times weekly.
10. The method of Clause 2, wherein the IL-21 composition is administered comcomitantly with the sorafenib composition.
11. The method of Clause 10, wherein the sorafenib composition is administered at 800 mg daily.
12. A method of treating metastatic melanoma comprising co-administering to a patient a composition comprising IL-21 polypeptide and a composition comprising a tyrosine kinase inhibitor.
13. A method of treating metastatic melanoma comprising co-administering to a patient a composition comprising IL-21 polypeptide and a composition comprising sutinitib.
14. A method of treating metastatic melanoma comprising co-administering to a patient a composition comprising IL-21 polypeptide and a composition comprising sorafenib.
15. The method of Clause 12, wherein the IL-21 composition is administered on a 5/9/5 schedule until disease progression.
16. The method of Clause 12, wherein the IL-21 composition is administered from 1 to 3 times weekly.

## Claims

1. A composition comprising IL-21 polypeptide and a composition comprising a tyrosine kinase inhibitor, for use in treating cancer selected from renal cell carcinoma, metastatic melanoma, or hepatocellular carcinoma, wherein said use comprises co-administration of said compositions to a patient.

2. Compositions for use according to Claim 1, wherein the cancer is renal cell carcinoma.

3. Compositions for use according to Claim 2, wherein the tyrosine kinase inhibitor is selected from the group consisting of Axitinib; Imatinib mesylate (Gleevec®); Gefitinib (Iressa®); Erlotinib hydrochloride (Tarceva®); Vandetanib (Zactima®), Tipifarnib (Zamestra®); Dasatinib (Sprycel®); Lonafarnib (Sarasar®); Vatalanib succinate; Lapatinib (Tykerb®);; Nilotinib; Lestaurtinib; Pazopanib hydrochloride; Canertinib dihydrochloride; Pelitinib; Tandutinib; Bosutinib; Semaxanib; AZD-2171; VX-680; EXEL-0999; ARRY-142886; PD-0325901; AMG-706 ; BIBF-1120; CP-547632; AEE-788; BMS-582664; JNK-401; R-788; AZD-1152 HQPA; NM-3; CP-868596; BMS-599626; PTC-299; ABT-869; EXEL-2880; AG-024322; XL-820; OSI-930; XL-184; KRN-951; CP-724714; E-7080; HKI-272; CHIR-258; ZK-304709; EXEL-7647; BAY-57-9352; BIBW-2992; AV-412; YN-968D1; Midostaurin; Perifosine; AG-024322; AZD-1152; ON-01910Na; and AZD-0530.

4. Compositions for use according to any of Claims 1-3, wherein in use the IL-21 composition is administered on a 5/9/5 schedule until disease progression.

5. Compositions for use according to any previous claim, wherein in use the IL-21 composition is administered from 1 to 3 times weekly.

6. Compositions for use according to Claim 1, wherein the cancer is metastatic melanoma.

7. Compositions for use according to Claim 6, wherein the tyrosine kinase inhibitor is selected from the group consisting of lmatinib mesylate (Gleevec®); Gefitinib (Iressa®); Erlotinib hydrochloride (Tarceva®); Vandetanib (Zactima®), Tipifarnib (Zarnestra®); Dasatinib (Sprycel®); Lonafarnib (Sarasar®); Vatalanib succinate; Lapatinib (Tykerb®); Axitinib; Nilotinib; Lestaurtinib; Pazopanib hydrochloride; Canertinib dihydrochloride; Pelitinib; Tandutinib; Bosutinib; Semaxanib; AZD-2171; VX-680; EXEL-0999; ARRY-142886; PD-0325901; AMG-706 ; BIBF-1120; CP-547632; AEE-788; BMS-582664; JNK-401; R-788; AZD-1152 HQPA; NM-3; CP-868596; BMS-599626; PTC-299; ABT-869; EXEL-2880; AG-024322; XL-820; OSI-930; XL-184; KRN-951; CP-724714; E-7080; HKI-272; CHIR-258; ZK-304709; EXEL-7647; BAY-57-9352; BIBW-2992; AV-412; YN-968D1; Midostaurin; Perifosine; AG-024322; AZD-1152; ON-01910Na; and AZD-0530.

8. Compositions for use according to Claim 6 or 7, wherein in use the IL-21 composition is administered on a 5/9/5 schedule until disease progression.

9. Compositions for use according to any of Claims 6-8, wherein in use the IL-21 composition is administered from 1 to 3 times weekly.

10. Compositions for use according to Claim 1, wherein the cancer is hepatocellular carcinoma.

11. Compositions for use according to Claim 10, wherein the tyrosine kinase inhibitor is selected from the group consisting of Erlotinib hydrochloride (Tarceva®); AZD-2171; BMS-582664; Lapatinib (Tykerb®); Gefitinib (Iressa®); lmatinib mesylate (Gleevec®); Vandetanib (Zactima®), Tipifarnib (Zamestra®); Dasatinib (Sprycel®); Lonafamib (Sarasar®); Axitinib; Vatalanib succinate;; Nilotinib; Lestaurtinib; Pazopanib hydrochloride; Canertinib dihydrochloride; Pelitinib; Tandutinib; Bosutinib; Semaxanib;; VX-680; EXEL-0999; ARRY-142886; PD-0325901; AMG-706 ; BIBF-1120; CP-547632; AEE-788;; JNK-401; R-788; AZD-1152 HQPA; NM-3; CP-868596; BMS-599626; PTC-299; ABT-869; EXEL-2880; AG-024322; XL-820; OSI-930; XL-184; KRN-951; CP-724714; E-7080; HKI-272; CHIR-258; ZK-304709; EXEL-7647; BAY-57-9352; BIBW-2992; AV-412; YN-968D1; Midostaurin; Perifosine; AG-024322; AZD-1152; ON-01910N; and AZD-0530.

12. Compositions for use according to Claim 10 or 11, wherein in use the IL-21 composition is administered on a 5/9/5 schedule until disease progression.

13. Compositions for use according to Claims 10-12, wherein in use the IL-21 composition is administered from 1 to 3 times weekly.

14. Use of a composition comprising IL-21 polypeptide and a composition comprising a tyrosine kinase inhibitor for the manufacture of a medicament for co-administration of said compositions to a patient for treating renal cell carcinoma, metastatic melanoma, or hepatocellular carcinoma.
